(19) Europäisches Patentamt
European Patent Office
Office européen des brevets

(11) **EP 3 669 838 A1**

(12) **EUROPEAN PATENT APPLICATION**

(43) Date of publication:
**24.06.2020 Bulletin 2020/26**

(51) Int Cl.:
*A61F 13/00* (2006.01)　　　*A61F 13/02* (2006.01)

(21) Application number: **18215494.8**

(22) Date of filing: **21.12.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Paul Hartmann S.A.
67730 Châtenois (FR)**

(72) Inventors:
• **MERCKEL, Fabien
67100 Straßbourg (FR)**
• **THIEBAUT, Renaud
67730 Chatenois (FR)**

(74) Representative: **Matzdorf, Thorsten et al
Paul Hartmann AG
Patents & Licensing
Paul-Hartmann-Straße 12
89522 Heidenheim (DE)**

(54) **ARRAY OF ABSORBENT DRESSINGS FOR THE TREATMENT OF WOUNDS**

(57)　　Array for the treatment of wounds comprising a first absorbent wound dressing 10, 30) that is non-adhesive and a second absorbent wound dressing (20, 40) that is adhesive, wherein the first wound dressing (10, 30) comprising a first absorbent pad 12, 32) comprising superabsorbent fibers and/or particles, and wherein the second wound dressing (20, 40) comprises a second absorbent pad (22, 42) comprising superabsorbent fibers and/or particles, characterized in that the second wound dressing (20, 40) comprises means for the adhesive fixation of the second wound dressing (20, 40) to the skin of a patient, wherein the means for the adhesive fixation comprise a skin-friendly silicone adhesive (23b, 43b).

Figure 1

**Description**

The present invention deals with an array of absorbent wound dressings, in particular for the treatment of wounds with moderate to high levels of exudate.

For the treatment of chronic or acute wounds producing moderate to high levels of exudate a well-accepted treatment is the treatment with absorbent dressings comprising superabsorbent materials. Several wound dressings are available on the market addressing the needs of these kind of wounds. Wound dressings of this kind usually comprise an absorbent body comprising superabsorbent materials. Some manufacturers of superabsorbent wound dressings provide arrays of wound dressings, wherein each part of the array comprises the same kind of absorbent body and wherein each part of the array has a different size or shape in order to provide an array of different wound dressings suitable for the treatment of the same kind of wounds in different wound sizes and/or at specific parts of the patient's body. However, individual patients suffering from the same kind of wound not only differ in the size or location of the wound but also in the characteristics in wound surrounding skin. For example, the wound surrounding skin can exhibit different sensitivity dependent on its characteristics. Intact skin is less sensitive than macerated skin or fragile skin known as parchment skin. If the wound is treated with a dressing that is appropriate for the treatment of the wound, but not for the skin surrounding the wound due to its sensitivity characteristics, severe discomfort, harm and pain can be caused by the dressing. Therefore, the sensitivity of the wound surrounding skin should be carefully taken into consideration for the choice of the appropriate wound dressing.

Therefore, there is a need for arrays of wound dressings comprising different wound dressings that are commonly suitable for the treatment of a certain wound but differ in the capability to address specific needs of individual patients.

An array according to claim 1 solves this problem.

The present invention deals with an array of first and second wound dressings, each of the dressings is suitable for the treatment of the same type of wound but for individual patients possessing different types of skin surrounding the wound. Wounds that can be treated appropriately with wound dressings of an array according to the present invention include wounds exhibiting moderate to high levels of exudate. These wounds can be acute wounds as well as chronic wounds. Including but not limiting examples are burns, wounds after operation, trauma wounds, venous ulcer, pressure ulcer, diabetic foot ulcer, tumor wounds. The skin surrounding such a wound can have different characteristics. It can be intact, injured, macerated or dry and fragile. Individual patients suffering from the same wound can differ significantly in the characteristics of the wound surround skin. Because of the very often long lasting treatment time, the characteristics of wound surrounding skin can change in an individual patient during the duration of treatment of the wound. Therefore, an array according to the present invention particularly is suitable to treat patients suffering from a certain wound whose characteristics of wound surrounding skin change during the duration of treatment.

An array of at least first and second wound dressings is any arrangement of the at least first and second wound dressings. Such arrangement or combination can occur as first and second packages, the first package containing the first wound dressings and the second package containing the second wound dressings. Alternatively, such arrangement or combination can occur as a kit containing the at least first and second wound dressings in a common packaging. An array of first and second wound dressings might or might not be indicated under similar trademarks or brands. The first and second wound dressings can contain at least one common element to be recognizable as being parts of the same array. Common elements can be words, significant parts of words, common color, similar type of writing, or any other possibility to indicate products as being part of the same array. An array can occur as a common offer for sale of the at least first and second wound dressings as complementary, for example in a store, in a product catalogue or in an internet offer.

An array according to this invention comprises at least two different absorbent wound dressings, the first absorbent wound dressing is non-adhesive, and the second wound dressing is adhesive, wherein the second wound dressing comprises adhesive means comprising a skin-friendly silicone adhesive.

An array comprising at least one non-adhesive absorbent dressing and at least one adhesive absorbent dressing has the advantage to provide appropriate dressings for the treatment of moderately to highly exuding wounds for patient suffering from the same wounds but having different types of skin surrounding the wound. In particular, such an array is advantageous if one patient suffering from a certain wound at the beginning of treatment has intact skin surrounding the wound and during treatment develops a skin surrounding the wound that is macerated or fragile.

In the present application the terms proximal side or proximal surface are to be understood as that side or surface of a layer or material of the wound dressing that is in use facing a wound. The terms distal side or distal surface are to be understood as that side or surface of a layer or material that is in use facing away from the wound.

A superabsorbent material is generally understood to be a water-insoluble, swellable polymer that can absorb a multiple of its own weight of liquid such as water, saline solutions, or body fluids. The absorption of liquid results in the formation of a hydrogel. The absorption capacity for pure water is typically greater than the absorption capacity for saline liquid. In connection with this invention, the term superabsorbent material refers, in particular, to a material exhibiting a w value (free swell capacity) according to the standard test method WSP 240.2 (05) of at least 10 g/g, preferably at least 20 g/g. The test method WSP 240.2 (05) for determining the w value is described in "Standard Test Methods for the Nonwovens and Related Industries," 2008 edition (published by "EDANA, International Association Serving the Nonwovens and Related Industries," Cary, N.C., U.S.A. and "INDA, Association of the Nonwovens Fabrics Industry," Brussels, Belgium).

The first wound dressing is non-adhesive and comprises an absorbent pad.

The first wound dressing is non-adhesive. That is, the dressing does not comprise any means for adhesive fixation of the dressing to the skin of a patient. This kind of dressing is advantageous for patients having skin surrounding the wound that is sensitive to contact with an adhesive. For example, macerated skin or fragile skin, so-called parchment skin, would adversely affected by contact with an adhesive. This kind of dressing should be fixed by a secondary dressing to the skin of the patient. A suitable secondary dressing is a fixation bandage.

The first dressing comprises a first absorbent pad comprising superabsorbent fibers and/or particles. Wound dressings comprising superabsorbent fibers and/or particles have been recognized as advantageous for wounds with moderate to high levels of exudate.

The absorbent pad of the first dressing can comprise further absorbent materials, e.g absorbent fibers. Absorbent fibers cause a quick absorption of wound exudate while superabsorbent materials exhibit a high absorption capacity. Suitable absorbent fibers can be cellulose-derived materials. In a preferred embodiment the absorbent fibers consist essentially of cellulose fibers. Suitable superabsorbent materials can be in the form of particles, gels or fibers. If the superabsorbent material is in the form of fibers, the fibers can be loosely distributed within the absorbent fibers or can be in a textile material. The textile material can comprise solely superabsorbent fibers or can further comprise absorbent fibers. The textile material can be in the form of any textile material that is suitable for incorporation into a wound dressing, e.g. nonwovens, woven, warp-knitted or weft-knitted materials.

The absorbent pad of the first dressing preferably comprises an absorbent core and an envelope surrounding the absorbent core.

The envelope of the first dressing is any kind of material enveloping the absorbent core. In one embodiment, the envelope comprises a sheet layer that is wrapped around the absorbent core.

In a further embodiment, the envelope surrounding the absorbent core of the first dressing comprises a first layer that covers the proximal side of the absorbent core facing the wound in use, and a second layer that covers the distal side of the absorbent core facing away from the wound in use. The first layer of the absorbent core extends over the proximal side of the absorbent core and the second layer of the absorbent core extends over the distal side of the absorbent core, each of them leaving a border area surrounding the absorbent core. The first and the second layer of the envelope of the absorbent pad are joined to each other along the border area surrounding the absorbent core.

The first sheet layer of the envelope of the first dressing must be water-permeable to provide access of wound exudate to the absorbent core.

The second sheet layer of the envelope of the first dressing can be water-permeable or water-impermeable. If it is water-impermeable, leakage of wound exudate from the dressing can be prevented.

The first and the second sheet layer of the envelope of the first dressing can comprise any suitable material including textile materials or polymer foils or films, particularly nonwoven material, woven materials, warp-knitted textiles, weft-knitted textiles, perforated or non-perforated foils, perforated or non-perforated films.

The absorbent core of the first dressing has a proximal side and a distal side. The absorbent core comprises an absorbent material. An absorbent is capable of absorbing liquids such as wound exudate. The material can comprise any wound exudate-absorbing material, particularly absorbent fibers and/or absorbent particles.

Suitably, this material comprises a mixture of absorbent fibers and absorbent particles.

Suitable materials for absorbent fibers can be cellulose or cellulose-based polymers, viscose, polyester, polyamide, derivatives, copolymers and mixtures thereof. Furthermore, suitable fibers include superabsorbent fibers that can be based on polyacrylic acid, sodium polyacrylate, polyacrylic acid esters, polyacrylamide, polyvinyl alcohol, copolymers and mixtures thereof. A preferred material is cellulose.

Suitable materials for absorbent particles include, but are not limited to, superabsorbent particles. Suitable materials for superabsorbent particles include, but are not limited to, polyacrylic acid, sodium polyacrylate, polyacrylic acid esters, copolymers and mixtures thereof. A preferred material is sodium polyacrylate.

In a preferred embodiment, the absorbent core of the first dressing comprises a mixture of absorbent fibers consisting essentially of cellulose and superabsorbent particles made from polyacrylic acid and sodium polyacrylate. Such an absorbent core exhibits rapid absorption of wound exudate and rapid distribution within the absorbent core.

The superabsorbent material can be present in an amount of from 1% by weight to 99% by weight within the absorbent core. Preferably, the amount of superabsorbent material comprises from 30-55% by weight of the absorbent core.

The absorbent pad of the first dressing further can comprise a diffusion layer. This layer enables an easier distribution of wound exudate in a horizontal manner within the absorbent core. This layer can be present on the proximal side of the absorbent core between the proximal layer of the envelope and the proximal surface of the absorbent core.

The diffusion layer can also be present as a layer of material that envelopes the entire absorbent core. Preferably, the diffusion layer is a tissue surrounding the layer of absorbent material. In a preferred embodiment, this tissue is made from cellulose and has a weight of 15 to 20 g/m$^2$.

The envelope of the first dressing comprises a first layer of a material exhibiting hydrophilic properties that is liquid-permeable. The material can comprise a material that exhibits hydrophilic properties by its chemical nature or can comprise a material originally exhibiting hydrophobic properties that is treated by a chemical or physical process to exhibit hydrophilic properties. Suitable processes for the hydrophilization of originally non-hydrophilic materials include electrochemical processes, flame treatment, corona treatment and plasma treatment. Suitable materials can be cellulose, polyester, polyamide, polyethylene, polypropylene, or copolymers from two or more of the materials. Preferably, the first layer of hydrophilic material comprises either a polypropylene nonwoven that has been treated physically in order to exhibit hydrophilic properties or it comprises a nonwoven comprising a mixture of viscose and polyamide fibers. Preferred materials can be a nonwoven comprising 55 % Polyamide and 45 % viscose fibers having a weight of 37 g/m$^2$ purchased under the name M1526 from the company Freudenberg (Germany) or a nonwoven comprising 63 % polypropylene and 37 % viscose fibers having a weight of 45 g/m$^2$ purchased under the name 670532/2 from the company Freudenberg (Germany).

The envelope of the first dressing comprises a second layer of a material exhibiting hydrophobic properties that is liquid-impermeable. The material can exhibit hydrophobic properties by its chemical nature or can comprise material originally exhibiting hydrophilic properties that is provided with hydrophobic properties by chemical or physical treatment, e.g. by the treatment with fluorocarbons, silicones, alkanes etc.

Both layers of the envelope of the first dressing surrounding the absorbent core can be colored in any way that is suitable. Usually, medical products have a white color signaling cleanness and purity. In one embodiment, one or both layers have a slightly green color. This color provides a strong color contrast when wetted with wound exudate, enabling the nurse staff to immediately recognize when the absorption capacity of the wound dressing has been reached. Surprisingly it has been found that a green color similar to RAL6019 provides the strongest color contrast with wound exudate while simultaneously signaling cleanness and purity.

The first and the second layer of the envelope of the absorbent pad of the first wound dressing are joined to each other. This joining can be formed by any suitable process for the joining of materials, directly or indirectly.

In a preferred embodiment the first and the second layer of the envelope of the first dressing each comprise a material exhibiting thermoplastic properties. It is possible to join the first layer and the second layer along a joining line by a thermic process. Thermic processes for the joining of materials can be performed in a continuous manner and therefore can be more cost-effective than other procedures. Suitable thermic processes for the joining of materials include heat welding, laser welding and ultrasonic welding.

In a preferred embodiment the first and the second layer of the envelope of the first dressing surrounding the absorbent core are joined by a thermic process and therefore comprise a welding connection. A welding connection resulting from a thermic connection process exhibits a favorable connection characterized by its welding strength and its flexibility. Welding strength within the meaning of this application is the force that is necessary to separate two layers that are joined with each other along a joining line. Welding strength can be determined by a method described below in the example part of this application. In a preferred embodiment the welding strength is higher than 0.75 N/ 15 mm, preferably higher than 1 N/ 15 mm.

Flexibility within the meaning of this application is the property that enables a material to bend and to change its conformation. It is characterized by the stiffness. A high welding strength is advantageous to prevent undesired delamination of the joined materials. Delamination of the first and the second layer of the absorbent core even in a minor extent can cause leakage. In that case wound exudate that already has been absorbed within the absorbent core could diffuse in outer zones of the wound dressing and contact wound surrounding intact skin via passage through the perforations in the wound contacting layer or totally leaving the wound dressing. This can cause maceration of intact skin surrounding the wound area. Furthermore, a leakage of wound exudate would cause a weakening of the adhesive connection between the backing layer and the perforated wound contact layer. If the leaked wound exudate diffuses along the distal side of the absorbent core that can cause the formation of wrinkles in the backing material which causes a discomfortable feeling for the user.

Flexibility is important for the dressing to be able to adapt to irregular body surface. Furthermore, a non-flexible dressing is very stiff and causes pressure on the patient's body surface during movement resulting in discomfort or pain.

In a preferred embodiment the welding connection comprises at least one non-continuous welding line. A non-continuous welding line exhibits a flexibility which is favorable for the patient's comfort.

In a preferred embodiment, the welding connection comprises four to six parallel non-continuous welding lines resulting in a favorably balanced relationship of both properties welding strength and flexibility.

In a preferred embodiment the at least one welding line is arranged parallel to the machine direction of the manufacturing process.

In a preferred embodiment the second layer of the envelope of the first dressing comprises a thermoplastic material that is a hydrophobic material and the first layer of the envelope comprises a thermoplastic material that is the same material as in the first layer and that is treated chemically and/or physically to exhibit hydrophilic properties. If the first and the second layer comprises materials of different chemical nature, the physical properties of these materials can differ significantly. This can be disadvantageous in a connection process where conditions must be found that are compatible for each of the materials. In a thermic connection process the temperature must be higher than the melting points of each material. However, thermically sensitive materials can degrade during this procedure if the process temperature is too high. Therefore, depending on the nature of the chemical materials of the first and the second layer there the process opportunities can be strongly limited. If the first and the second layer comprise a material that has the same chemical nature, the materials do not differ in their physical properties relevant for a connection procedure. This is especially advantageous in a thermic connection process, when the process temperature only must be slightly above the softening temperature of the material, and the risk for thermical degradation of the material is significantly reduced.

In a preferred embodiment the material of the first layer and of the second layer of the envelope of the first dressing both is polypropylene.

The first wound dressing can comprise a further wound contact layer that is non-adhesive.

In a preferred embodiment the wound contact layer of the first dressing has an open area of between 10 to 25 % of the entire area of the wound contact layer. Surprisingly it has been found that an open area in this range results in a favorable absorption velocity of wound exudate. Absorption velocity within the meaning of this application means the time of the absorption of wound exudate. It can be determined by a test method described in the example part of this application.

The wound contact layer of the first dressing comprises apertures for the passage of wound exudate to the absorbent core. Apertures can have any geometrical shape, regular or irregular, in particular a circular, oval, triangular, square, rectangular, pentangular, hexagonal or other polygonal shape with uniform or non-uniform side length.

In a preferred embodiment the wound contact layer of the first dressing comprises apertures of any of these shapes having an average open area of from 0,03 $mm^2$ to 7,0 $mm^2$.

In a preferred embodiment the wound contact layer of the first dressing comprises apertures having an essentially circular shape and having an average diameter of between 0,2 mm to 3,0 mm. Surprisingly it has been found that the absorption velocity is favorable for a wound contact layer comprising apertures having a circular shape and an average diameter of 2,2 mm to 2,8 mm.

Suitably, the open area of the aperture layer is from about 10 % to 30 %. Surprisingly it has been found that the absorption velocity is favorable for a wound contact layer having an open area of 11 % to 22%, preferably 12 % to 18 %. Suitably, the density of the apertures is from about 1000 to 100000 apertures per $m^2$, for example about 5000 to 50000 apertures per $m^2$.

In a preferred embodiment the absorbent core of the first dressing exhibits an absorption capacity of at least 100 g/ 100 $cm^2$, preferably more than 120 g/ 100 $cm^2$, more preferably more than 130 g/ 100 $cm^2$ according to the test method described within the example part of this application.

In a preferred embodiment the absorbent core of the first dressing comprises a mixture of absorbent fibers and superabsorbent materials. Absorbent fibers cause a quick absorption of wound exudate while superabsorbent materials exhibit a high absorption capacity. Suitable absorbent fibers can be cellulose-derived materials. In a preferred embodiment the absorbent fibers consist essentially of cellulose fibers. Suitable superabsorbent materials can be in the form of particles, gels or fibers. If the superabsorbent material is in the form of fibers, the fibers can be loosely distributed within the absorbent fibers or can be in a textile material. The textile material can comprise solely superabsorbent fibers or can further comprise absorbent fibers. The textile material can be in the form of any textile material that is suitable for incorporation into a wound dressing, e.g. nonwovens, woven, warp-knitted or weft-knitted materials.

In a preferred embodiment the joining line of the first dressing has an inner end and an outer end, and that the envelope comprises a space between the first and the second layer of the envelope, wherein the absorbent core is placed, the space being limited by the inner end of the joining line, and the space having an area $A_1$, the absorbent core having an area $A_2$, wherein the area $A_2$ is at least 80 % of the area $A_1$.

The first wound dressing can comprise a backing layer that is arranged on the distal surface of the absorbent pad.

The backing layer of the first dressing may have any shape, such as square, rectangular, circular, oval, trapezium-shaped, suitably with rounded corners.

The backing layer of the first dressing supports the absorbent pad and provides a barrier to passage of microorganisms through the dressing. The backing layer is substantially liquid-impermeable but permeable for water vapor. The backing layer suitably has a moisture vapor transmission rate (MVTR) from 300 to 30000 g/ $m^2$/ 24h, suitably 1000 to 15000 g/ $m^2$/ 24h, and in one embodiment 1000 to 5000 g/ $m^2$/ 24h according to test method EN 13726. The backing layer has a thickness of 10 $\mu$m to 100 $\mu$m, suitably 12 $\mu$m to 75 $\mu$m, and in a preferred embodiment 15 $\mu$m to 50 $\mu$m, most preferred 25 $\mu$m to 35 $\mu$m. The backing layer comprises a proximal surface and a distal surface. The distal surface of the backing layer suitably is a low-friction surface in order to exhibit advantageous wearing properties without sticking to clothes or causing noise during use.

Suitable polymers for forming the backing layer of the first dressing material include polyurethanes, poly alkoxyalkyl acrylates and methyl acrylates such as those disclosed in GB1280631. Suitably, the backing layer comprises a continuous layer of a high density blocked polyurethane foam that is predominantly closed-cell. A suitable backing layer material is the polyurethane film available as film number 1305 from the company Coveris, having a thickness of 30 μm.

Preferably, the backing layer of the first dressing is transparent to have a view on the absorbent pad positioned on the proximal side of the backing layer.

The backing layer of the first dressing can be coated with an adhesive on the proximal surface. The adhesive secures the backing layer to further layers of the wound dressing, particularly to the absorbent pad and to the wound contact layer. The backing layer can be coated with adhesive continuously, that is the adhesive covers the entire proximal surface of the backing layer. In different embodiments, the backing layer can be coated partially with adhesive. In these embodiments the adhesive can be in the form of stripes, dots or in different patterns. In a further embodiment the backing layer comprises an adhesive layer on the proximal surface that leaves an adhesive-free part in the center of the backing layer to reduce contact with the distal surface of the absorbent pad. The MVTR in an adhesive-free region of the backing layer is increased. If the absorbent core within the absorbent pad swells after absorption of wound exudate the absorbent pad changes its spatial extension. This can either cause delamination due to the occurrence of shearing forces within the wound dressing, or it can cause stress and pain on the wound and/or the wound surrounding skin due to shearing forces acting on the wound and skin of the patient. A dressing leaving an adhesive-free area between the distal surface of the absorbent pad and the proximal surface of the backing layer can be advantageous in this respect.

The adhesive preferably is a pressure-sensitive adhesive of the type that is conventionally used for medical dressings. Suitable pressure-sensitive adhesives can be based on acrylic acid, acrylate ester copolymers, polyvinyl ethyl ether and polyurethane such as those described in GB1280631. The basis weight of the pressure-sensitive adhesive is suitably 10 g/ $m^2$ to 100 g/ $m^2$, preferably 15 g/ $m^2$ to 50 g/ $m^2$, more preferably 20 g/ $m^2$ to 30 g/ $m^2$.

The first wound dressing, the absorbent core of the first wound dressing, the absorbent pad of the first wound dressing, the envelope of the first wound dressing and a wound contact layer of the first wound dressing each can have any suitable size and basic geometrical shape, particularly squares, rectangles, circles, ovals, polygons. If the basic geometrical shape of any of these layers comprises corners, these corners preferably are rounded. This reduces the risk of curling of the layer and of delamination from other layers or skin. In a preferred embodiment, the radius for the rounded corners is from 5 mm to 15 mm, more preferably 12,5 mm.

The second wound dressing is adhesive. That is, the dressing comprises means for adhesive fixation of the dressing to the skin of a patient. An adhesive wound dressing is easy to apply to a patient's wound because of its self-adhesiveness. It does not need any secondary fixation, and therefore it's use is very efficient. Suitable means for adhesive fixation includes, but are not limited to, an adhesive wound contact layer or an adhesive border surrounding the absorbent pad. In the present invention, the means for the adhesive fixation comprise a skin-friendly silicone adhesive. It has been shown that this kind of adhesive means is suitable for efficient but atraumatic fixation of a dressing.

The second wound dressing is an adhesive wound dressing comprising an absorbent pad. The absorbent pad of the second wound dressing comprises superabsorbent fibers and/or particles.

The second dressing comprises a second absorbent pad comprising superabsorbent fibers and/or particles. Wound dressings comprising superabsorbent fibers and/or particles have been recognized as advantageous for wounds with moderate to high levels of exudate.

Superabsorbent materials exhibit a high absorption capacity. They can absorb an amount of water that is a multiple of its own weight. For the present application, 1 g of a superabsorbent material is capable of absorbing at least 50 g of distilled water. Suitable superabsorbent materials can be in the form of particles, gels or fibers.

The absorbent pad of the second dressing can comprise further absorbent materials, e. g absorbent fibers. Absorbent fibers cause a quick absorption of wound exudate while superabsorbent materials exhibit a high absorption capacity. Suitable absorbent fibers can be cellulose-derived materials. In a preferred embodiment the absorbent fibers consist essentially of cellulose fibers. Suitable superabsorbent materials can be in the form of particles, gels or fibers. If the superabsorbent material is in the form of fibers, the fibers can be loosely distributed within the absorbent fibers or can be in a textile material. The textile material can comprise solely superabsorbent fibers or can further comprise absorbent fibers. The textile material can be in the form of any textile material that is suitable for incorporation into a wound dressing, e.g. nonwovens, woven, warp-knitted or weft-knitted materials.

The absorbent pad of the second dressing preferably comprises an absorbent core and an envelope surrounding the absorbent core.

The envelope of the second dressing is any kind of material enveloping the absorbent core. In one embodiment, the envelope comprises a sheet layer that is wrapped around the absorbent core. In a further embodiment, the envelope surrounding the absorbent core of the second dressing comprises a first layer that covers the proximal side of the absorbent core facing the wound in use, and a second layer that covers the distal side of the absorbent core facing away from the wound in use. The first layer of the absorbent core extends over the proximal side of the absorbent core and the second layer of the absorbent core extends over the distal side of the absorbent core, each of them leaving a border area surrounding the absorbent core. The first and the second layer of the envelope of the absorbent pad are joined to each other along the border area surrounding the absorbent core.

The first sheet layer of the envelope of the second dressing must be water-permeable in order to provide access of wound exudate to the absorbent core.

The second sheet layer of the envelope of the second dressing can be water-permeable or water-impermeable. If it is water-impermeable, leakage of wound exudate from the dressing can be prevented.

The first and the second sheet layer of the envelope of the second dressing can comprise any suitable material including textile materials or polymer foils or films, particularly nonwoven material, woven materials, warp-knitted textiles, weft-knitted textiles, perforated or non-perforated foils, perforated or non-perforated films.

In a further embodiment the second wound dressing comprises a backing layer, an absorbent pad and a wound contact layer.

The backing layer of the second dressing may have any shape, such as square, rectangular, circular, oval, trapezium-shaped, suitably with rounded corners.

The backing layer of the second dressing supports the absorbent pad and provides a barrier to passage of microorganisms through the dressing. The backing layer is substantially liquid-impermeable but permeable for water vapor. The backing layer suitably has a moisture vapor transmission rate (MVTR) from 300 to 30000 g/ $m^2$/ 24h, suitably 1000 to 15000 g/ $m^2$/ 24h, and in one embodiment 1000 to 5000 g/ $m^2$ /24h according to test method EN 13726. The backing layer has a thickness of 10 $\mu$m to 100 $\mu$m, suitably 12 $\mu$m to 75 $\mu$m, and in a preferred embodiment 15 $\mu$m to 50 $\mu$m, most preferred 25 $\mu$m to 35 $\mu$m. The backing layer comprises a proximal surface and a distal surface. The distal surface of the backing layer suitably is a low-friction surface in order to exhibit advantageous wearing properties without sticking to clothes or causing noise during use.

Suitable polymers for forming the backing layer of the second dressing material include polyurethanes, poly alkoxyalkyl acrylates and methyl acrylates such as those disclosed in GB1280631. Suitably, the backing layer comprises a continuous layer of a high density blocked polyurethane foam that is predominantly closed-cell. A suitable backing layer material is the polyurethane film available as film number 1305 from the company Coveris, having a thickness of 30 $\mu$m.

Preferably, the backing layer of the second dressing is transparent to have a view on the absorbent pad positioned on the proximal side of the backing layer.

The backing layer of the second dressing can be coated with an adhesive on the proximal surface. The adhesive secures the backing layer to further layers of the wound dressing, particularly to the absorbent pad and to the wound contact layer. The backing layer can be coated with adhesive continuously, i.e. the adhesive covers the entire proximal surface of the backing layer. In different embodiments, the backing layer can be coated partially with adhesive. In these embodiments the adhesive can be in the form of stripes, dots or in different patterns. In a further embodiment the backing layer comprises an adhesive layer on the proximal surface that leaves an adhesive-free part in the center of the backing layer to reduce contact with the distal surface of the absorbent pad. The MVTR in an adhesive-free region of the backing layer is increased. If the absorbent core within the absorbent pad swells after absorption of wound exudate the absorbent pad changes its spatial extension. This can either cause delamination due to the occurrence of shearing forces within the wound dressing, or it can cause stress and pain on the wound and/or the wound surrounding skin due to shearing forces acting on the wound and skin of the patient. A dressing leaving an adhesive-free area between the distal surface of the absorbent pad and the proximal surface of the backing layer can be advantageous in this respect.

The adhesive preferably is a pressure-sensitive adhesive of the type that is conventionally used for medical dressings. Suitable pressure-sensitive adhesives can be based on acrylic acid, acrylate ester copolymers, polyvinyl ethyl ether and polyurethane such as those described in GB1280631. The basis weight of the pressure-sensitive adhesive is suitably 10 g/ $m^2$ to 100 g/ $m^2$, preferably 15 g/ $m^2$ to 50 g/ $m^2$, more preferably 20 g/ $m^2$ to 30 g/ $m^2$

In a further embodiment the absorbent pad of the second dressing comprises an absorbent core and an envelope surrounding the absorbent core.

The absorbent core of the second dressing has a proximal side and a distal side. The absorbent core comprises an absorbent material. An absorbent is capable of absorbing liquids such as wound exudate. The material can comprise any wound exudate-absorbing material, particularly absorbent fibers and/or absorbent particles.

Suitably, the material comprises a mixture of absorbent fibers and absorbent particles.

Suitable materials for absorbent fibers can be cellulose or cellulose-based polymers, viscose, polyester, polyamide, derivatives, copolymers and mixtures thereof. Furthermore, suitable fibers include superabsorbent fibers that can be based on polyacrylic acid, sodium polyacrylate, polyacrylic acid esters, polyacrylic amide, polyvinyl alcohol, copolymers and mixtures thereof. A preferred material is cellulose.

Suitable materials for absorbent particles include, but are not limited to, superabsorbent particles. Suitable materials for superabsorbent particles include, but are not limited to, polyacrylic acid, sodium polyacrylate, polyacrylic acid esters, copolymers and mixtures thereof. A preferred material is sodium polyacrylate.

In a preferred embodiment, the absorbent core of the second dressing comprises a mixture of absorbent fibers consisting essentially of cellulose and superabsorbent particles made from polyacrylic acid and sodium polyacrylate. Such an absorbent core exhibits rapid absorption of wound exudate and rapid distribution within the absorbent core.

The superabsorbent material can be present in an amount of from 1 % by weight to 99 % by weight within the absorbent core. Preferably, the amount of superabsorbent material comprises from 30 % to 55 % by weight of the absorbent core.

The absorbent pad further can comprise a diffusion layer. This layer enables an easier distribution of wound exudate in a horizontal manner within the absorbent core. This layer can be present on the proximal side of the absorbent core between the proximal layer of the envelope and the proximal surface of the absorbent core. The diffusion layer can also be present as a layer of material that envelopes the entire absorbent core. Preferably, the diffusion layer is a tissue surrounding the layer of absorbent material. In a preferred embodiment, this tissue is made from cellulose and has a weight of 15 g/ $m^2$ to 20 g/ $m^2$.

In a preferred embodiment, the envelope of the second dressing comprises a first, proximal layer of a first liquid-permeable material and a second, distal layer of a material different from the material of the first layer.

The envelope surrounding the absorbent core of the second dressing comprises a first layer that covers the proximal side of the absorbent core facing the wound in use, and a second layer that covers the distal side of the absorbent core facing away from the wound in use. The first layer of the absorbent core extends over the proximal side of the absorbent core and the second layer of the absorbent core extends over the distal side of the absorbent core, each of them forming a border area surrounding the absorbent core. The first and the second layer of the envelope of the absorbent pad are joined to each other by a joining line along the border area surrounding the absorbent core.

The envelope of the second dressing comprises a first layer of a material exhibiting hydrophilic properties that is liquid-permeable. The material can comprise a material that exhibits hydrophilic properties by its chemical nature or can comprise a material originally exhibiting hydrophobic properties that is treated by a chemical or physical process to exhibit hydrophilic properties. Suitable processes for the hydrophilization of originally non-hydrophilic materials include electrochemical processes, flame treatment, corona treatment and plasma treatment. Suitable materials can be cellulose, polyester, polyamide, polyethylene, polypropylene, or copolymers from two or more of the aforementioned materials. Preferably, the first layer of hydrophilic material comprises either a polypropylene nonwoven that has been treated physically in order to exhibit hydrophilic properties or it comprises a nonwoven comprising a mixture of viscose and polyamide fibers. Preferred materials can be a nonwoven comprising 55 % Polyamide and 45 % viscose fibers having a weight of 37 g/ $m^2$ purchased under the name M1526 from the company Freudenberg (Germany) or a nonwoven comprising 63 % polypropylene and 37 % viscose fibers having a weight of 45 g/ $m^2$ purchased under the name 670532/2 from the company Freudenberg (Germany).

The envelope of the second dressing comprises a second layer of a material exhibiting hydrophobic properties that is liquid-impermeable. The material can exhibit hydrophobic properties by its chemical nature or can comprise material originally exhibiting hydrophilic properties that is provided with hydrophobic properties by chemical or physical treatment, e.g. by the treatment with fluorocarbons, silicones, alkanes etc.

Both layers of the envelope of the second dressing surrounding the absorbent core can be colored in any way that is suitable. Usually, medical products have a white color signaling cleanness and purity. In one embodiment, one or both layers have a slightly green color. This color provides a strong color contrast when wetted with wound exudate, enabling the nurse staff to immediately recognize when the absorption capacity of the wound dressing has been reached. Surprisingly it has been found that a green color similar to RAL6019 provides the strongest color contrast with wound exudate while simultaneously signaling cleanness and purity.

The first and the second layer of the envelope of the absorbent pad of the second dressing are joined to each other. This joining can be formed by any suitable process for the joining of materials.

In a preferred embodiment the first and the second layer of the envelope of the second dressing each comprise a material exhibiting thermoplastic properties. It is possible to join the first layer and the second layer along a joining line by a thermic process. Thermic processes for the joining of materials can be performed in a continuous manner and therefore can be more cost-effective than other procedures. Suitable thermic processes for the joining of materials include heat welding, laser welding and ultrasonic welding.

In a preferred embodiment the first and the second layer of the envelope of the second dressing surrounding the absorbent core are joined by a thermic process and therefore comprise a welding connection. A welding connection resulting from a thermic connection process exhibits a favorable connection characterized by its welding strength and its flexibility. Welding strength within the meaning of this application is the force that is necessary to separate two layers that are joined with each other along a joining line. Welding strength can be determined by a method described below in the example part of this application. In a preferred embodiment the welding strength is higher than 0.75 N/ 15 mm, preferably higher than 1 N/ 15 mm.

A high welding strength is advantageous to prevent undesired delamination of the joined materials. Delamination of the first and the second layer of the absorbent core even in a minor extent can cause leakage. In that case wound exudate that already has been absorbed within the absorbent core could diffuse in outer zones of the wound dressing and contact wound surrounding intact skin via passage through the perforations in the wound contacting layer or totally leaving the wound dressing. This can cause maceration of intact skin surrounding the wound area. Furthermore, a leakage of wound exudate would cause a weakening of the adhesive connection between the backing layer and the perforated wound contact layer. If the leaked wound exudate diffuses along the distal side of the absorbent core that can cause the formation of wrinkles in the backing material which causes a discomfortable feeling for the user.

Flexibility within the meaning of this application is the property that enables a material to bend and to change its conformation. It is characterized by the stiffness.

Flexibility is important for the dressing to be able to adapt to irregular body surface. Furthermore, a non-flexible dressing is very stiff and causes pressure on the patient's body surface during movement resulting in discomfort or pain.

In a preferred embodiment the welding connection of the second dressing comprises at least one non-continuous welding line. A non-continuous welding line exhibits a flexibility which is favorable for the patient's comfort.

In a preferred embodiment, the welding connection of the second dressing comprises four to six parallel non-continuous welding lines resulting in a favorably balanced relationship of both properties welding strength and flexibility.

In a preferred embodiment the at least one welding line of the second dressing is arranged parallel to the machine direction of the manufacturing process.

In a preferred embodiment the second layer of the envelope of the second dressing comprises a thermoplastic material that is a hydrophobic material and the first layer of the envelope comprises a thermoplastic material that is the same material as in the first layer and that is treated chemically and/or physically to exhibit hydrophilic properties. If the first and the second layer comprises materials of different chemical nature, the physical properties of these materials can differ significantly. This can be disadvantageous in a joining process where conditions must be found that are compatible for each of the materials. In a thermic joining process the temperature must be higher than the melting points of each material. However, thermically sensitive materials can degrade during this procedure if the process temperature is too high. Therefore, depending on the nature of the chemical materials of the first and the second layer there the process opportunities can be strongly limited. If the first and the second layer comprise a material that has the same chemical nature, the materials do not differ in their physical properties relevant for a connection procedure. This is especially advantageous in a thermic joining process, when the process temperature only has to be slightly above the softening temperature of the material, and the risk for thermical degradation of the material is significantly reduced.

In a preferred embodiment the material of the first layer and of the second layer both is Polypropylene.

In a preferred embodiment the wound contact layer has an open area of between 10 to 25% of the entire area of the wound contact layer. Surprisingly it has been found that an open area in this range results in a favorable absorption velocity of wound exudate. Absorption velocity within the meaning of this application means the time of the absorption of wound exudate. It can be determined by a test method described in the example part of this application.

The wound contact layer of the second dressing comprises a layer of skin-friendly silicone adhesive. Suitably, the silicone composition is a so-called soft skin adhesive silicone elastomer. The total coating weight of the silicone is suitably from about 15 g/ m$^2$ to about 500 g/ m$^2$, preferably 50 g/ m$^2$ to 250 g/ m$^2$, more preferably 100 g/ m$^2$ to 200 g/ m$^2$.

A proper wound dressing must adhere to the skin which allows a use of the dressing for several hours, preferably for at least one day, more preferably for at least three days, most preferably for up to seven days without losing adhesiveness. On the other hand, the dressing must exhibit skin-friendly properties that allow the dressing to be removed without causing pain to the patient or irritation to the wound or the surrounding skin. Surprisingly, it has been found that a second dressing exhibiting a peel value on skin of between 200 mN/ cm, preferably 350 mN /cm and 650 mN/ cm is advantageous in this manner.

Although it is possible to provide a wound contact layer consisting essentially of a soft skin adhesive silicone elastomer, the wound contact layer of the second dressing preferably comprises a further layer of a perforated sheet material wherein the perforations of the sheet material correspond to the apertures of the silicone adhesive without being occluded by the silicone adhesive. The perforated sheet material may be any medically acceptable perforated sheet material, including textile materials. Suitably, the perforated sheet material is a unitary sheet material such as a polymer mesh or an aperture polymer film. Suitable polymer materials include polyethylene, polypropylene, polyester, polyvinyl acetate, ethylene vinyl acetate and polyurethane. Suitably, the sheet material has a thickness of 1 μm to 100mm, preferably 5 μm to 50 μm. A preferred sheet material is a polyurethane material purchased as Acrysil® covered with 150 g/ m$^2$ silicone adhesive from the company Zodiac (France). A further preferred sheet material is a knitted fabric impregnated with a skin-friendly silicone adhesive. A knitted textile comprises meshes forming apertures. These apertures preferably can have different sizes. A preferred textile material comprises a first fraction of meshes having a diameter of significantly less than 1mm and a second fraction of meshes having a diameter of significantly more than 1mm. In a preferred embodiment, the textile material is impregnated with a skin-friendly silicone adhesive occluding only the first fraction of meshes but leaving open the second fraction of meshes.

The wound contact layer of the second dressing comprises apertures for the passage of wound exudate to the absorbent core. Apertures can have any geometrical shape, regular or irregular, in particular a circular, oval, triangular, square, rectangular, pentangular, hexagonal or other polygonal shape with uniform or non-uniform side length.

In a preferred embodiment the wound contact layer of the second dressing comprises apertures of any of these shapes having an average open area of from 0,03 mm$^2$ to 7,0 mm$^2$.

In a preferred embodiment the wound contact layer of the second dressing comprises apertures having an essentially circular shape and having an average diameter of between 0,2 mm to 3,0 mm. Surprisingly it has been found that the absorption velocity is favorable for a wound contact layer comprising apertures having a circular shape and an average diameter of from 2,2 mm to 2,8 mm.

Suitably, the open area of the aperture layer is from about 10 % to 30 %. Surprisingly it has been found that the absorption velocity is favorable for a wound contact layer having an open area of 11 % to 22 %, preferably 12 % to 18 %. Suitably, the density of the apertures is from about 1000 to 100000 apertures per m$^2$, for example about 5000 to 50000 apertures per m$^2$.

In the second wound dressing, the backing layer, the absorbent pad and the wound contact layer can have different or similar extensions.

In one embodiment the second dressing comprises a backing layer and an absorbent pad having a proximal and a distal surface, wherein the distal surface of the absorbent pad is covered by the backing layer, and the backing layer has an extension in both area directions that is larger than the area of the distal surface of the absorbent pad, while the absorbent pad and the wound contact layer are co-extensive. The backing layer therefore forms a border area completely surrounding the absorbent pad. The surrounding border area can be covered with a skin-friendly adhesive to build a dressing having a central absorbent pad and an adhesive border. This type of dressing is called an island-type dressing.

In another embodiment the second dressing comprises a backing layer and an absorbent pad having a proximal and a distal surface, wherein the distal surface of the absorbent pad is covered by the backing layer, and the backing layer has an extension in both area directions that is larger than the area of the distal surface of the absorbent pad, while the wound contact layer and the backing layer are co-extensive. This type of dressing is called a sandwich-type dressing. The latter type of dressing has a lower risk for delamination.

In a preferred embodiment the absorbent core of the second dressing exhibits an absorption capacity of at least 100 g/ 100 cm$^2$, preferably more than 120 g/ 100 cm$^2$, more preferably more than 130 g/ 100 cm$^2$ according to the test method described within the example part of this application.

In a preferred embodiment the absorbent core of the second dressing comprises a mixture of absorbent fibers and superabsorbent materials. Absorbent fibers cause a quick absorption of wound exudate while superabsorbent materials exhibit a high absorption capacity. Suitable absorbent fibers can be cellulose-derived materials. In a preferred embodiment the absorbent fibers consist essentially of cellulose fibers. Suitable superabsorbent materials can be in the form of particles, gels or fibers. If the superabsorbent material is in the form of fibers, the fibers can be loosely distributed within the absorbent fibers or can be in a textile material. The textile material can comprise solely superabsorbent fibers or can further comprise absorbent fibers. The textile material can be in the form of any textile material that is suitable for incorporation into a wound dressing, e.g. nonwovens, woven, warp-knitted or weft-knitted materials.

The wound dressing, the backing layer, the absorbent core, the absorbent pad, the envelope and the wound contact layer each can have any suitable size and basic geometrical shape, particularly squares, rectangles, circles, ovals, polygons. If the basic geometrical shape of any of these layers comprises corners, these corners preferably are rounded. This reduces the risk of curling of the layer and of delamination from other layers or skin. In a preferred embodiment, the radius for the rounded corners is from 5 mm to 15 mm, more preferably 12,5 mm.

In a preferred embodiment the joining line of the second dressing has an inner end and an outer end, and that the envelope comprises a space between the first and the second layer of the envelope, wherein the absorbent core is placed, the space being limited by the inner end of the joinig line, and the space having an area $A_1$, the absorbent core having an area $A_2$, wherein the area $A_2$ is at least 80 % of the area $A_1$.

The second dressing of an array according to the present invention may further comprise at least one removable cover sheet to cover the adhesive areas on the proximal surface of the wound dressing. The cover sheet covers and protects the absorbent pad and prevents premature adhesion of the adhesive parts of the wound dressing. It may comprise a film of polyethylene, polypropylene or fluorocarbons and paper coated with these materials or silicone. A suitable material for a cover sheet can be a polyethylene foil having a thickness of $100\mu$m and can be purchased from the company Flextrus®.

In a preferred embodiment an array comprises a first wound dressing having an absorbent core formed from an airlaid mixture of cellulose and a superabsorbent polymer based on sodium polyacrylate particles, the airlaid is wrapped by a cellulose tissue. The first wound dressing of this array further comprises an envelope. This envelope comprises a proximal sheet layer made from a nonwoven of polyamide and viscose fibers. This envelope further comprises a distal sheet layer made from a nonwoven of polypropylene fibers. This distal sheet layer is water-impermeable but permeable to air. Both sheet layers of the envelope extend over the absorbent core and are joined by a welding line formed by ultrasonic welding. This first wound dressing does not comprise any means for adhesive fixation to the skin of a patient. In this embodiment the array comprises a second wound dressing having an absorbent core, an envelope and an adhesive wound contact layer. The absorbent core of the second dressing is formed from an airlaid mixture of cellulose and a superabsorbent polymer based on sodium polyacrylate particles, the airlaid is wrapped by a cellulose tissue. The envelope of the second wound dressing of this array comprises a proximal sheet layer made from a nonwoven comprising a mixture of polyamide and viscose fibers. The envelope of the second wound dressing of this array comprises a distal sheet layer made from a nonwoven comprising polypropylene fibers. This distal sheet layer is water-impermeable but permeable to air. Both sheet layers of the envelope extend over the absorbent core and are joined by a welding line formed by ultrasonic welding. This second wound dressing of this array further comprises a wound contact layer that is coextensive with the proximal surface of the envelope of the absorbent core. This wound contact layer is a knitted fabric from polyethylene terephthalate impregnated with a skin-friendly silicone gel adhesive. This second wound dressing further comprises a two-part removable cover sheet.

In an alternatively preferred embodiment an array comprises a first wound dressing having an absorbent core formed from an airlaid mixture of cellulose and a superabsorbent polymer based on sodium polyacrylate particles, the airlaid is wrapped by a cellulose tissue. The first wound dressing of this array further comprises an envelope. This envelope comprises a proximal sheet layer made from a nonwoven of polyamide and viscose fibers. This envelope further comprises a distal sheet layer made from a nonwoven of polypropylene fibers. This distal sheet layer is water-impermeable but permeable to air. Both sheet layers of the envelope extend over the absorbent core and are joined by a welding line formed by ultrasonic welding. This first wound dressing does not comprise any means for adhesive fixation to the skin of a patient. In this embodiment the array comprises a second wound dressing having a backing layer, an absorbent core, an envelope and an adhesive wound contact layer. The backing layer is a water-impermeable but vapor-permeable polyurethane film material. This backing layer is coated on its proximal side with an acrylic adhesive and has an extension in both area directions that is larger than the area of the distal surface of the envelope of the absorbent core. The backing layer therefore forms a border area completely surrounding the absorbent pad. The absorbent core of the second dressing is formed from an airlaid mixture of cellulose and a superabsorbent polymer based on sodium polyacrylate particles, the airlaid is wrapped by a cellulose tissue. The envelope of the second wound dressing of this array comprises a proximal sheet layer made from a nonwoven comprising a mixture of polyamide and viscose fibers. The envelope of the second wound dressing of this array comprises a distal sheet layer made from a nonwoven comprising poly propylene fibers. This distal sheet layer is water-impermeable but permeable to air. Both sheet layers of the envelope extend over the absorbent core and are joined by a welding line formed by ultrasonic welding. This second wound dressing of this array further comprises a wound contact layer that is coextensive with the proximal surface of the backing layer. This wound contact layer is a layer of a knitted textile material made from polyethylene terephthalate coated with a layer of a skin-friendly silicone gel adhesive. This textile material comprises a first fraction of meshes having a diameter of significantly less than 1mm and a second fraction of meshes having a diameter of significantly more than 1mm. In a preferred embodiment, the textile material is impregnated with a skin-friendly silicone adhesive occluding only the first fraction of meshes but leaving open the second fraction of meshes. The non-occluded meshes of the second fraction of meshes correspond to the apertures of the silicone adhesive without being occluded by the silicone adhesive. This second wound dressing further comprises a two-part removable cover sheet.

Figure 1 shows an array according to the present invention comprising a first wound dressing and a second wound dressing.

Figure 2 shows an alternative array according to the present invention comprising a first wound dressing and a second wound dressing.

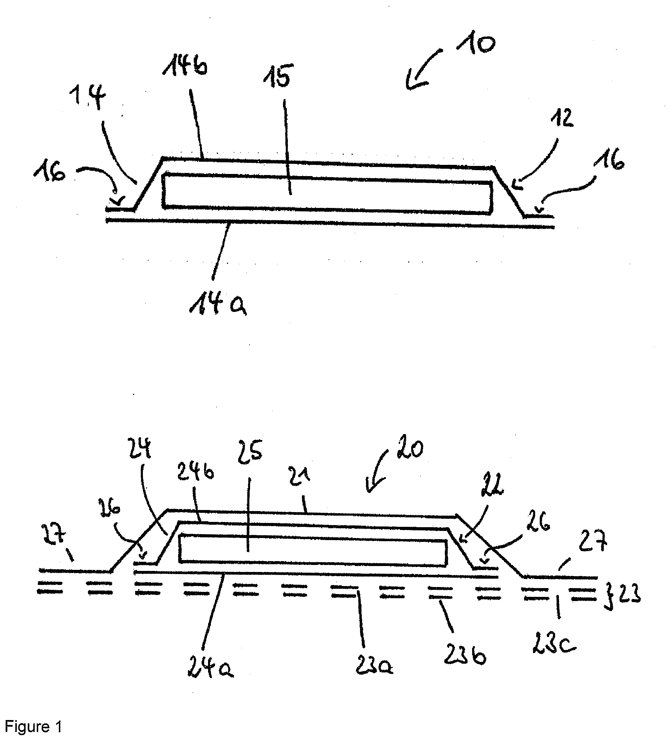

Figure 1 shows an array of a first wound dressing (10) and a second wound dressing (20). The first wound dressing (10) comprises an absorbent core (15) and an envelope (14). The envelope (14) consists of a proximal sheet layer (14a) and a distal sheet layer (14b). The proximal sheet layer (14a) is made from a nonwoven comprising a mixture of polyamide fibers and viscose fibers and is water-permeable. The distal sheet layer (14b) is made from a nonwoven consisting of polypropylene fibers. The distal sheet layer (14b) is substantially water-impermeable, but permeable to air. The absorbent core (15) comprises an airlaid mixture of cellulose fibers and superabsorbent particles based on sodium polyacrylate. The second wound dressing (20) has an absorbent pad (22), a backing layer (21) and a wound contact layer (23). The absorbent pad (22) comprises an absorbent core (25) and an envelope (24). The envelope (24) is formed from a proximal layer (24a) and a distal layer (24b). The wound contact layer (23) is formed from a layer of a perforated sheet material (23a) and a layer of skin-friendly silicone adhesive (23b). The backing layer (21) is made from a vapour-permeable and substantially liquid-impermeable polyurethane film material with low friction characteristics having a thickness of 30μm. The absorbent core (25) comprises a mixture of cellulose fibers and superabsorbent sodium polyacrylate particles in a pre-fabricated airlaid material. The absorbent core (25) further comprises a diffusion layer in form of a sheet of cellulose tissue that is wrapped around the airlaid mixture of cellulose and polyacrylate particles. The proximal layer (24a) of the envelope (24) is a nonwoven made from a mixture of viscose fibers and polyamide fibers. The distal layer (24b) of the envelope (24) is a nonwoven made from polypropylene fibers. The proximal layer (24a) of the envelope (24) covers the proximal side of the absorbent core (25) and extends over the proximal side of the absorbent core (25), thereby forming a border area (26) surrounding the absorbent core (25). The distal layer (24b) of the envelope (24) covers the distal side of the absorbent core (25) and extends over the distal side of the absorbent core (25), thereby forming a border area (26) surrounding the absorbent core (25). The proximal layer (24a) of the envelope (24) and the distal layer (24b) of the envelope (24) are joined along the border area (26) surrounding the absorbent core (22). The perforated sheet material (23a) of the wound contact layer (23) is made from a polyurethane film that comprises apertures (23c) having a circular shape. These apertures (23c) have similar round shapes having an average diameter of 2,4 mm and are arranged in a regular pattern resulting in an open area of 15 %. The wound contact layer (23) further comprises a layer of skin-friendly silicone adhesive (23b). The backing layer (21) extends over the absorbent pad (22), thereby forming a border area (27) surrounding the absorbent pad (22). The wound contact layer (23) extends over the absorbent pad (22), thereby forming a border area (27) surrounding the absorbent pad (22). The backing layer (21) and the wound contact layer (23) are coextensive.

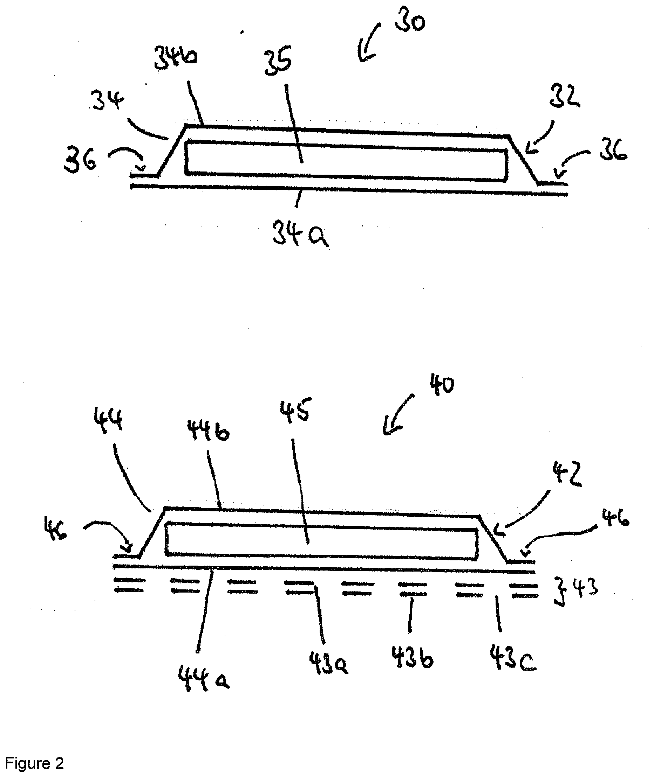

Figure 2 shows an array of a first wound dressing (30) and a second wound dressing (40). The first wound dressing (30) comprises an absorbent core (32) and an envelope (34). The envelope (34) consists of a proximal sheet layer (34a) and a distal sheet layer (34b). The proximal sheet layer (34a) is made from a nonwoven comprising a mixture of polyamide fibers and viscose fibers and is water-permeable. The distal sheet layer (34b) is made from a nonwoven consisting of polypropylene fibers. The distal sheet layer (34b) is substantially water-impermeable, but permeable to air. The absorbent core (32) comprises an airlaid mixture of cellulose fibers and superabsorbent particles based on sodium polyacrylate. The second wound dressing (40) has an absorbent core (42), an envelope (44) surrounding the absorbent core (42) and a wound contact layer (43). The envelope (44) is formed from a proximal layer (44a) and a distal layer (44b). The wound contact layer (43) is formed from a layer (43a) of a knitted textile made from polyethylene terephthalate impregnated with a layer of skin-friendly silicone adhesive (43b). This textile material (43a) comprises a first fraction of meshes having a diameter of significantly less than 1mm and a second fraction of meshes (43c) having a diameter of significantly more than 1mm. The textile material (43a) is impregnated with a skin-friendly silicone adhesive (43b) occluding only the first fraction of meshes but leaving open the second fraction (43c) of meshes. The non-occluded meshes of the second fraction of meshes (43c) correspond to the apertures (43c) of the silicone adhesive (43b) without being occluded by the silicone adhesive (43b). The absorbent core (42) comprises a mixture of cellulose fibers and superabsorbent sodium polyacrylate particles in a pre-fabricated airlaid material. The proximal layer (44a) of the envelope (44) is a nonwoven made from a mixture of viscose fibers and polyamide fibers. The distal layer (44b) of the envelope (44) is a nonwoven made from polypropylene fibers. The proximal layer (44a) of the envelope (44) covers the proximal side of the absorbent core (45) and extends over the proximal side of the absorbent core (45), thereby forming a border area (46) surrounding the absorbent core (45). The distal layer (44b) of the envelope (44) covers the distal side of the absorbent core (45) and extends over the distal side of the absorbent core (45), thereby forming a border area (46) surrounding the absorbent core (45). The proximal layer (44a) of the envelope (44) and the distal layer (44b) of the envelope (44) are joined along the border area (46) surrounding the absorbent core (42).

Examples

Example 1: Wound dressing

The wound dressing is a preferred second wound dressing of the array of the present invention. It has an absorbent pad, a backing layer and a wound contact layer. The absorbent pad comprises an absorbent core and an envelope. The envelope is formed from a proximal layer and a distal layer. The wound contact layer is formed from a layer of a perforated sheet material and a layer of skin-friendly silicone adhesive. The backing layer is made from a vapour-permeable and liquid-impermeable polyurethane film material with low friction characteristics having a thickness of 30 $\mu$m. The absorbent core comprises a mixture of cellulose fibers and superabsorbent sodium polyacrylate particles in a pre-fabricated airlaid material. The absorbent core further comprises a diffusion layer in form of a sheet of cellulose tissue that is wrapped around the airlaid mixture of cellulose fibers and polyacrylate particles. The proximal layer of the envelope is a nonwoven made from a mixture of viscose fibers and polyamide fibers. The distal layer of the envelope is a nonwoven made from polypropylene fibers. The perforated sheet material of the wound contact layer is made from a polyurethane film that comprises apertures having a circular shape. These apertures exhibit similar shapes having an average diameter of 2,4 mm and are arranged in a regular pattern resulting in an open area of 15 %. The wound contact layer further comprises a layer of skin-friendly silicone adhesive without occluding the apertures of the perforated sheet material.

Example 2: Test solutions used in the characterization of wound dressings

Solution A (saline solution)

2 L deionized water

0,74 g Calcium chloride dihydrate ($CaCl_2 \cdot 2\ H_2O$, CAS: 10035-04-5)

16,6 g Sodium chloride (NaCl, CAS: 7647-14-5)

Solution B (exudates solution)

1 L deionized water

70 g Albumin from chicken egg white (CAS: 9006-59-1)

0,2 g Allura Red AC (CAS: 25956-17-6)

9 g Sodium chloride (NaCl, N° CAS: 7647-14-5)

0,3 g Calcium chloride dihydrate ($CaCl_2 \cdot 2H_2O$, N° CAS: 10035-04-5)

2 g Methyl 4-hydroxybenzoate (CAS: 99-76-3)

1 g Propyl 4-hydroxybenzoate (CAS: 94-13-3)

Example 3: Test method absorption velocity

The absorption velocity of a dressing is determined by the time necessary to completely absorb a test solution. Test solutions can be either saline solution (solution A) or exudate solution (solution B). Solutions (saline solution or exudates solution) as well as test products have to be preconditioned at room temperature prior testing by leaving the test products and the solutions for two hours at 2 °C.

A 50 mL buret is filled with the test solution. The liquid level should be fixed at 15 mL.

The dressing is placed under the buret, that surface which in use faces the wound now faces the buret. The distance between the buret and the dressing is adjusted to 1 cm. The tap of the buret is opened, and simultaneously a stopwatch is started, while 2 mL of test solution are allowed to flow out. The stopwatch is stopped immediately when the 2 mL of test solution are completely absorbed by the dressing, i.e. when no drop is remaining over the perforation of the wound contact layer. If the dressing is big enough, 1 to 3 measurements can be made on the same dressing.

Positions of the tests on a dressing are one in the centre, two further points along a diagonal in direction to the corners of the dressing, similar to the position of the three points on a regular die.

Each value has to be classified in the following categories

| Time (s) | [0 to 4,9] | [5,0 to 10,9] | [11,0 to 30,9] | [31,0 to 60,0] | >60 |
|---|---|---|---|---|---|
| category | Immediate | Very good | Good | Average | Critical |

A dressing according to the invention has been compared with commercially available competitor dressings Biatain® silicone, Allevyn® Life, Mepilex® border (which is considered as being a dressing according to the disclosure of WO93/19709 and WO93/19710).

Absorption velocity:

| Dressing | Absorption velocity [percentage of samples] | | | | |
|---|---|---|---|---|---|
|  | immediate | Very quick | quick | average | slow |
| Example 1 | 20% | 80% | 0% | 0% | 0 % |
| Biatain® silicone | 0% | 0% | 0% | 20 % | 80% |
| Allevyn® Life | 0% | 0% | 100 % | 0% | 0% |
| Mepilex® border | 0% | 0% | 0% | 100 % | 0% |

Example 4: Test method absorption capacity

Solutions (saline solution or exudates solution) as well as test products have to be preconditioned at room temperature prior testing by leaving the test products and the test solutions for two hours at 22 °C.

The basic mass (mi) of a dressing is determined after removal of the release liners.

The length and width of the absorbent is determined so that the absorbent core surface (S) can be determined. A bowl is filled with the test solution. The weight of testing solution should be at least 40 times higher than the dressing itself. The dressing is put into the bowl, and simultaneously a stopwatch is started. That side of the dressing which in use faces the wound side should face the bottom of the bowl, the back side of the dressing should be on the top. The dressing should not be glued to the bottom of the bowl. The dressing is left within the the bowl for 30 min +/- 1 min. After 30 min, the dressing is removed from the bowl. The dressings should only be manipulated via the borders and not the absorbent core itself. The dressings are on 1 corner to a stand with a clamp and allowed to hang for 20 min at room temperature. The wet mass ($m_2$) of the dressing is determined. The amount of liquid ($m_{liquid}$) absorbed is calculated:

$$m_{liquid} = m_2 - m_1$$

The absorption capacity is the amount of liquid absorbed ($m_2$-$m_1$) by the absorbent core surface (S) and is given in g/ 100cm$^2$:

$$\text{absorption capacity} = m_2\text{-}m_1/S \times 100$$

A dressing according to the invention has been compared with commercially available competitor dressings Biatain® silicone, Allevyn® Life, Mepilex® border (which is considered as being a dressing according to the disclosure of WO93/19709 and WO93/19710).

Comparison of absorption capacities:

| Dressing | Absorption capacity in g/ 100 cm$^2$ |
|---|---|
| Example 1 | 147 |
| Biatain® silicone | 116 |
| Allevyn® Life | 87 |
| Mepilex® border | 54 |

Example 5: Test method for adhesiveness on skin - Peel test

Peel tests were performed on skin (forearm) with stripes similar to borders of full dressings. Peel tests were performed on immobile arms, with a start at 90 °. Tests were performed after 1min/ 5min/ 10min of wearing. Minimum samples tested is 3 for each testing point.

A dressing according to the invention has been compared with commercially available competitor dressings Biatain® silicone, Allevyn® Life, Mepilex® border (which is considered as being a dressing according to the disclosure of WO93/19709 and WO93/19710).

| Dressing | Adhesiveness on skin in N/ 20mm | | |
|---|---|---|---|
| | 1 min | 5 min | 10 min |
| Example 1 | 0,68 | 1,02 | 1,03 |
| Biatain® silicone | 0,96 | 1,84 | 1,86 |
| Mepilex® border | 1,01 | 1,02 | 1,03 |

Example 6: Test method for welding strength

The test for welding strength is carried out with Tensile Tester MTS C42.503E; cell strength 50 N.

15 x 25 mm samples are stamped in the absorbent pad, including the pad, at least at 2 mm from the corner. After stamping, the absorbent material is removed. The sample to be tested is only made of 2 nonwovens welded together. The clamp jaws of the tensile tester are placed in a way that the distance between the 2 jaws is 2 cm. Each nonwoven is placed in a separate jaw. The tensile tester is started at a speed of 200 mm/ min until the 2 nonwovens are separated. This can happen either after a complete break of the weld or after complete tearing of the nonwovens themselves.

The welding strength is the average strength over the testing period. It is given in N/ 15mm.

**Claims**

1. Array of wound dressings for the treatment of wounds comprising a first absorbent wound dressing 10, 30) that is non-adhesive and a second absorbent wound dressing (20, 40) that is adhesive, wherein the first wound dressing (10, 30) comprises a first absorbent pad 12, 32) comprising superabsorbent fibers and/or particles, and wherein the second wound dressing (20, 40) comprises a second absorbent pad (22, 42) comprising superabsorbent fibers and/or particles, **characterized in that** the second wound dressing (20, 40) comprises means for the adhesive fixation of the second wound dressing (20, 40) to the skin of a patient, wherein the means for the adhesive fixation comprise a skin-friendly silicone adhesive (23b, 43b).

2. Array according to claim 1, **characterized in that** the first wound dressing (10, 30) comprises a first absorbent pad (12, 32) comprising a first absorbent core (15, 35) and a first envelope (14, 34) surrounding the first absorbent core (15, 35), wherein the first absorbent core (15, 35) comprises superabsorbent fibers and/or particles, and wherein the second wound dressing (20, 40) comprises a second absorbent pad (22, 42) comprising a second absorbent core (25, 45) and a second envelope (24, 44) surrounding the second absorbent core (25, 45), wherein the second absorbent core (25, 45) comprises superabsorbent fibers and/or particles.

3. Array according to claim 2, **characterized in that** the first envelope (14, 34) of the first wound dressing (10, 30) comprises a first proximal sheet layer (14a, 34a) covering the proximal side of the first absorbent core (15, 35) and a first distal sheet layer (14b, 34b) covering the distal side of the first absorbent core (15, 35), and wherein the first proximal sheet layer (14a, 34a) extends over the proximal side of the first absorbent core (15, 35) and the first distal sheet layer (14b, 34b) extends over the distal side of the first absorbent core (15, 35), each of them forming a border area (16, 36) surrounding the first absorbent core (15, 35), the first proximal sheet layer (14a, 34a) and the first distal sheet layer (14b, 34b) of the envelope (14, 34) being joined to each other along the border area (16, 36) surrounding the first absorbent core (15, 35).

4. Array according to claim 3, **characterized in that** the first proximal sheet layer (14a, 34a) and the first distal sheet layer (14b, 34b) each comprise a nonwoven material.

5. Array according to claim 3 or 4, **characterized in that** the first distal sheet layer (14b, 34b) is substantially water-impermeable.

6. Array according to at least one of the claims 2 to 5, **characterized in that** the first absorbent core (15, 35) further comprises fibers and/or particles selected from cellulose, viscose, cellulose-based materials, polyamide, polyester, polyethylene, polypropylene, co-polymers or mixtures thereof.

7. Array according to at least one of the claims 2 to 6, **characterized in that** the second envelope (24, 44) of the second wound dressing (20, 40) comprises a second proximal sheet layer (24a, 44a) covering the proximal side of the second absorbent core (25, 45) and a second distal sheet layer (24b, 44b) covering the distal side of the second absorbent core (25, 45), and wherein the second proximal sheet layer (24b, 44b) extends over the proximal side of the second absorbent core (25, 45) and the second distal sheet layer (24b, 44b) extends over the distal side of the second absorbent core (25, 45), each of them forming a border area (26, 46) surrounding the second absorbent core (25, 45), the second proximal sheet layer (24a, 44a) and the second distal sheet layer (24b, 44b) of the envelope (24, 44) being joined to each other along the border area (26, 46) surrounding the second absorbent core (25, 45).

8. Array according to at least one of the claims 2 to 7, **characterized in that** the second proximal sheet layer (24a, 44a) and the second distal sheet layer (24b, 44b) each comprise a nonwoven material.

9. Array according to at least one of the claims 2 to 8, **characterized in that** the second distal sheet layer (24b, 44b) is substantially water-impermeable.

10. Array according to at least one of claims 2 to 9, **characterized in that** the second absorbent core (25, 45) further comprises fibers and/or particles selected from cellulose, viscose, cellulose-based materials, polyamide, polyester, polyethylene, polypropylene, co-polymers or mixtures thereof.

11. Array according to at least one of the preceding claims, **characterized in that** the second wound dressing (20) comprises a vapour-permeable and substantially liquid-impermeable backing layer (21).

**12.** Array according to claim 11, **characterized in that** the second absorbent pad (24) comprises a proximal surface and a distal surface and the backing layer (21) comprises a coating of a pressure-sensitive adhesive, and that the backing layer (21) extends over the distal surface of the second absorbent pad (24) forming an adhesive border area (27) that surrounds the absorbent pad (24).

**13.** Array according to at least one of the preceding claims, **characterized in that** the second wound dressing (20, 40) comprises an adhesive wound contact layer (23, 43).

**14.** Array according to at least one of the preceding claims, **characterized in that** the adhesive wound contact layer (23, 43) of the second wound dressing (20, 40) comprises an apertured layer of a skin-friendly silicone adhesive (23b, 43b).

**15.** Array according to at least one of the preceding claims, **characterized in that** the second wound dressing (20) comprises a wound contact layer (23) and a backing layer (21), wherein the wound contact layer (23) of the second wound dressing (20) is coextensive with the backing layer (21) of the second wound dressing (20).

Figure 1

Figure 2

Europäisches
Patentamt

European
Patent Office

Office européen
des brevets

# EUROPEAN SEARCH REPORT

Application Number

EP 18 21 5494

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2012/095419 A1 (RIESINGER BIRGIT [DE]) 19 April 2012 (2012-04-19) * abstract * * figures 1,5,6,11,13-15 * * paragraphs [0013], [0049], [0055] - [0058], [0088] - [0093], [0105], [0124], [0129], [0130] * * claims 41,50-53 * | 1-15 | INV. A61F13/00 A61F13/02 |
| A | US 2005/143697 A1 (RIESINGER BIRGIT [DE]) 30 June 2005 (2005-06-30) * figures 1,2,3a,4,9-13 * * paragraphs [0001] - [0004], [0010], [0014], [0015], [0074] * | 1-15 | |
| A | US 2018/125721 A1 (HOGGARTH ANDREW [GB] ET AL) 10 May 2018 (2018-05-10) * abstract * * figures 1-7 * * paragraphs [0010], [0037], [0039], [0045], [0058], [0062], [0067], [0077] - [0079], [0105] - [0113] * | 1-15 | |
| | | | TECHNICAL FIELDS SEARCHED (IPC) |
| A | US 2009/156974 A1 (TRUELSEN JENS HOG [DK] ET AL) 18 June 2009 (2009-06-18) * paragraphs [0017], [0024], [0027], [0044], [0049], [0052] - [0054] * * claims 1,3-5,10,11 * | 1-15 | A61F |

-/--

The present search report has been drawn up for all claims

| Place of search | Date of completion of the search | Examiner |
|---|---|---|
| Munich | 7 May 2019 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 18 21 5494

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "Rieger Erste Hilfe Verband-Mix 64 Teile (60 Pflaster, 2 Wundpflaster, 1 Heftpflaster, 1 Mullbinde)", , 7 November 2018 (2018-11-07), XP055586381, Retrieved from the Internet: URL:https://www.amazon.de/Rieger-Verband-Mix-Wundpflaster-Heftpflaster-Mullbinde/dp/B07KB416KB/ref=sr_1_3?__mk_de_DE=ÅMÅZÕN&keywords=erste+hilfe+pflaster+mix&qid=1557233283&s=drugstore&sr=1-3 [retrieved on 2019-05-07] * the whole document * ----- | 1-15 | |

| | | TECHNICAL FIELDS SEARCHED (IPC) |
|---|---|---|

The present search report has been drawn up for all claims

| Place of search | Date of completion of the search | Examiner |
|---|---|---|
| Munich | 7 May 2019 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 21 5494

This annex lists the patent family members relating to the patent documents cited in the above-mentioned European search report.
The members are as contained in the European Patent Office EDP file on
The European Patent Office is in no way liable for these particulars which are merely given for the purpose of information.

07-05-2019

| Patent document cited in search report | Publication date | Patent family member(s) | Publication date |
|---|---|---|---|
| US 2012095419 A1 | 19-04-2012 | DE 102008034363 A1 | 05-02-2009 |
| | | DK 2178573 T3 | 03-09-2012 |
| | | DK 2481385 T3 | 19-01-2015 |
| | | EP 2178573 A2 | 28-04-2010 |
| | | EP 2481385 A1 | 01-08-2012 |
| | | EP 2805701 A1 | 26-11-2014 |
| | | US 2012095419 A1 | 19-04-2012 |
| | | WO 2009019224 A2 | 12-02-2009 |
| US 2005143697 A1 | 30-06-2005 | AT 8817 U2 | 15-01-2007 |
| | | AT 8890 U2 | 15-02-2007 |
| | | AU 2003233309 A1 | 11-11-2003 |
| | | CN 1652735 A | 10-08-2005 |
| | | DE 20207356 U1 | 12-06-2003 |
| | | DK 1507498 T3 | 23-11-2009 |
| | | DK 200700048 U3 | 13-04-2007 |
| | | EP 1507498 A1 | 23-02-2005 |
| | | EP 1994916 A2 | 26-11-2008 |
| | | ES 2331568 T3 | 08-01-2010 |
| | | FI 7594 U1 | 09-08-2007 |
| | | PL 206143 B1 | 30-07-2010 |
| | | PT 1507498 E | 22-10-2009 |
| | | US 2005143697 A1 | 30-06-2005 |
| | | WO 03094813 A1 | 20-11-2003 |
| US 2018125721 A1 | 10-05-2018 | AU 2016254448 A1 | 16-11-2017 |
| | | BR 112017023112 A2 | 10-07-2018 |
| | | CA 2983906 A1 | 03-11-2016 |
| | | CN 107995858 A | 04-05-2018 |
| | | EP 3288510 A1 | 07-03-2018 |
| | | GB 2537840 A | 02-11-2016 |
| | | JP 2018514360 A | 07-06-2018 |
| | | KR 20180129609 A | 05-12-2018 |
| | | US 2018125721 A1 | 10-05-2018 |
| | | WO 2016174399 A1 | 03-11-2016 |
| US 2009156974 A1 | 18-06-2009 | EP 1919412 A1 | 14-05-2008 |
| | | US 2009156974 A1 | 18-06-2009 |
| | | WO 2007025546 A1 | 08-03-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1280631 A
- WO 9319709 A
- WO 9319710 A

**Non-patent literature cited in the description**

- **CARY.** Standard Test Methods for the Nonwovens and Related Industries. EDANA, International Association Serving the Nonwovens and Related Industries, 2008